# FASCICULE DE BREVET EUROPEEN

(11) **EP 0 721 499 B1**
(45) Date de publication et mention de la délivrance du brevet: **30.09.1998**
(21) Numéro de dépôt: 94922287.1
(22) Date de dépôt: 21.07.1994
(51) Int. Cl.: C12N 1/26, B09B 3/00, C02F 3/34, A62D 3/00

(54) **PROCEDE DE REHABILITATION DE SOLS CONTAMINES PAR HYDROCARBURES ET AUTRES SUBSTANCES BIODEGRADABLES**
VERFAHREN ZUR BODENVERBESSERUNG DURCH VON KOHLENWASSERSTOFFEN UND ANDEREN BIOABBAUBAREN VERBINDUNGEN KONTAMINIERTEN BÖDEN
METHOD FOR THE REHABILITATION OF SOIL CONTAMINATED BY HYDROCARBONS AND OTHER BIODEGRADABLE SUBSTANCES

(30) Priorité: 01.09.1993 FR 9310416
(43) Date de publication de la demande: 17.07.1996
(73) Titulaire: CECA S.A., F-92091 Paris la Défense (FR)
(72) Inventeur: SCHULZ, Christian, F-64110 Gélos (FR)
(74) Mandataire: Haicour, Philippe
(86) Numéro de dépôt international: FR9400913
(87) Numéro de publication internationale: WO9506715

(56) Documents cités:
- EP-A- 0 550 023
- WO-A-91/19039
- WO-A-93/24612
- DE-A- 4 014 854
- GB-A- 2 084 608
- US-A- 3 883 397
- US-A- 3 959 127

## Description

La présente invention est du domaine de la réhabilitation des sols pollués. Elle concerne plus particulièrement un mode de traitement des sols industriels pollués par des hydrocarbures et autres composés organiques biodégradables.

L'élimination des hydrocarbures contenus dans les sols est essentiellement le fait de bactéries aérobies naturellement présentes dans la terre et qui trouvent l'unique source de leur nourriture hydrocarbonée dans ces hydrocarbures. La biodégradation des hydrocarbures est donc un phénomène naturel dont la vitesse dépend de beaucoup de facteurs, dont principalement la concentration des hydrocarbones et les caractéristiques de leur chaîne hydrocarbonée, mais aussi des autres nutriments dont les micro organismes ont besoin pour accomplir leurs fonctions vitales.

Dans les terres, où le carbone se trouve en excès par suite d'une pollution, les bactéries consomment, durant les phases de fermentation aérobie active, 15 à 30 fois plus de carbone que d'azote, selon que l'hydrocarbure est plus ou moins biodégradable. Les facteurs limitants de la biodégradation sont alors les nutriments en déficit, l'azote pour l'essentiel, puis le phosphore parmi les éléments moyens, et à un degré moindre le soufre et le potassium. Il est possible de limiter cet effet par apport des éléments déficitaires de façon à rétablir les conditions optimales pour le fonctionnement bactérien que l'on s'accorde à fixer en rapports pondéraux à :
- C/N = 20 à 70
- N/P = 2 à 10
- C/P = 75 à 150
- C/S = 100 à 300

Les compléments nutritionnels nécessaires pour l'invention sont ceux qui sont apportés en agriculture avec les engrais chimiques traditionnels. Aussi a-t-on préconisé d'utiliser pour la biodécontamination des engrais complets tels que présents sur le marché ou des formulations similaires, et de les appliquer selon des façons apparentées aux façons culturales. Comme les processus de dégradation des hydrocarbures sont sensiblement plus longs que ceux qui président à l'assimilation végétale chez les végétaux supérieurs, il a été fait recours de préférence à des formulations du type engrais retard. On trouve la description d'une telle application dans la demande internationale WO 91/19039 (Grace-Sierra Hort. Prod. Co). L'efficacité de ce procédé reste néanmoins limitée parce que la pollution à laquelle on s'attaque est celle d'un hydrocarbure qui n'est généralement pas uniformément réparti dans le sol contaminé, et que la diffusion du nutriment secondaire que l'on apporte sous la forme hydrophile d'un engrais retard n'établit pas en tous points les conditions recherchées d'une biodégradation totale.

La présente invention remédie à cet inconvénient en associant, dans un procédé de réhabilitation de sol contaminé par des hydrocarbures et autres produits biodégradables, un traitement mixte qui comprend l'apport d'une source de nutriments pour micro-organismes sous une double forme oléophile et hydrophile et d'un agent d'aération du sol.

La source de nutriments secondaires pour micro-organismes sous forme oléophile est empruntée à des microémulsions de substances nutritives de type N/P en solution aqueuse dans une phase externe constituée par un solvant organique lipomiscible. Ces microémulsions sont décrites dans le brevet français n° 80 20178 publié sous le n°2.490.672 (S.N.E.A. P) et son certificat d'addition n° 81 16626 publié sous le n° 2.512.057.

La source de nutriments secondaires sous forme hydrophile est un engrais à diffusion programmée du type N/P/K, ou plus simplement de type N/P/O parce que l'apport en potassium n'est en général pas nécessaire dans les conditions de la biodégradation recherchée. Pour mettre plus aisément l'invention en application, selon des règles qui sont exposées plus loin, il est préférable de disposer d'au moins deux formulations, par exemple d'un 18/1/0 et d'un 15/7/0. Les engrais courants sont formulés sous forme retard pour éviter leur entraînement trop rapide par les eaux des précipitations. Il n'y a que la diffusion naturelle des nutriments pour assurer l'établissement des conditions idéales de répartition locale des nutriments secondaires vers les points où la pollution hydrocarbonée est fixée. Il est donc souhaitable d'avoir une répartition aussi fine que possible des grains de nutriments hydrophiles dans le sol, ce qui impose une taille de leurs grains plus réduite que celle des engrais ordinaires. Alors que ceux-ci ont couramment 30 granules/gramme, les nutriments hydrophiles recommandés pour l'invention comprendront de 100 à 200 granules/gramme. Au surplus, il seront fortement colorés, de façon à visualiser leur distribution au cours de leur épandage et leur incorporation dans les sols à traiter.

L'agent d'aération du sol est destiné à faciliter un processus de décontamination par voie bactérienne qui fait appel à des bactéries aérobies. On utilise à cet effet des copeaux ou de la sciure de bois, de la rafle ou de la paille. La paille, notamment la paille des graminées céréales, est particulièrement intéressarte, parce que c'est un matériau hydrophobe ; la propriété en était exploitée dans la constitution des toits de chaume. On a constaté que lorsque de la paille était malaxée avec un sol contaminé avec des hydrocarbures, elle fixait une partie des hydrocarbures qui se trouvaient ainsi placés dans des conditions favorables à leur biodégradation aérobie, à savoir les tubes creux de la paille. Ce mécanisme est probablement amélioré en présence des tensioactifs apportés par la microémulsion utilisée comme nutriment sous forme hydrophobe. La rapidité et le résultat final du processus de décontamination s'en trouvent considérablement améliorés.

Pour la mise en oeuvre du procédé, la première opération à effectuer est une reconnaissance de la présence d'une flore bactérienne adéquate (Pseudomonas Cichorii, Rhodococcus Fascians, Arthrobactus divers, etc.). Cette reconnaissance s'effectue grâce à la réalisation d'un test bactérien consistant en une numération bactérienne par culture en milieu solide aérobie en boîte de Pétri. On procède ensuite au dosage du sol en carbone, azote et phosphore, et à une analyse, au moins sommaire, des hydrocarbures présents en aliphatiques, dont la durée de dégradation de l'ordre de 6 mois est relativement rapide, et aromatiques dont la durée de dégradation est nettement plus longue (8 à 12 mois pour les aromatiques, 16 mois pour les polycycliques). A partir de ces données, on détermine les dosages en nutriments bactériens oléophile et hydrophile pour amener la teneur globale du sol traité en C/N/P aux valeurs optimales voisines de 100/20/1. Comme base de départ, on compte sur une répartition nutriment oléophile/nutriment hydrophile de 10/90 en relevant d'autant plus ce rapport que les hydrocarbures contiennent plus d'aromatiques et/ou de polycycliques.

Pour le traitement du terrain, on commence par un épandage à la lance ou une pulvérisation du nutriment oléophile, utilisé préférentiellement sous une forme concentrée. Lorsque la contamination hydrocarbonée est intense, on abandonne le chantier à lui-même jusqu'à ce que de couleur noire initiale, le sol passe au brun ou au brun jaune. Cette mutation demande communément de 2 à 7 jours. On reprend alors le chantier en répandant sur le sol la paille, à un dosage de 0,1 % à 5 %, dépendant des caractéristiques physiques du sol, et le nutriment hydrophile en grains au dosage déterminé, puis on procède à un malaxage intime à l'aide de tout engin convenable, agricole ou de chantier. Avec des sols moyennement contaminés, l'épandage du nutriment oléophile et de la paille associée au nutriment hydrophile est réalisé dans un même temps. On peut abandonner le sol en l'état, ou encore l'excaver et le stocker en andains selon l'utilisation qui doit être faite des espaces traités. Le processus biologique se développe sur des durées variables, généralement de six à 18 mois, selon l'intensité de la pollution et la nature des hydrocarbures polluants. On suit la progression de la biodécontamination par prélèvements réguliers et dosage des hydrocarbures. Les terres sont considérées comme décontaminées lorsque leur teneur en hydrocarbures est ramenée à des valeurs, mesurées selon la norme AFNOR NFT 90 114, inférieures à 500 mg/kg (seuil à la date de la demande de brevet, modifiable pour s'accorder à la réglementation en vigueur).

### Exemple 1

Une zone délimitée dans l'enceinte d'un site industriel désaffecté était contaminée par des graisses moteur et des huiles de vidange. Le dosage moyen des hydrocarbures donnait une teneur de 62 g/kg de terre. Le test de présence des bactéries aérobies était positif, avec une flore totale aérobie de 4 10⁷ bactéries par gramme de sol. On a prévu et appliqué les conditions de traitement suivant :
- le nutriment oléophile, le produit commercialisé par CECA S.A. sous le nom d'INIPOL ® EAP22 qui est une microémulsion de N/P 10/1 de densité 0,996 environ, à raison de 100 g/m² ;
- paille, à répandre à raison de 1 % en volume ;
- le nutriment hydrophile, une composition N/P/K 15/7/O, commercialisée par CECA S.A. sous le nom d'INIPOL ® SP2, sous forme de billes de couleur vert foncé (200 granules/gramme) protégées par une couche antidiffusion, à incorporer à raison de 1000 g/m².

Le nutriment oléophile a été répandu à l'aide d'un pulvérisateur à la surface du sol, et le site a été laissé en l'état durant huit jours. On a ensuite répandu manuellement la paille (1% en volume) et les grains de nutriment hydrophile. Un malaxage a été opéré à la pelle mécanique avec trois reprises successives des terres jusqu'à l'obtention d'un mélange homogène. Le matériau a été alors excavé et disposé en andains d'environ 5 mètres de large et 25 mètres de long sur une hauteur de 1,5 mètre, disposés sur une aire protégée par une géomembrane. On a suivi l'évolution de la teneur en hydrocarbures (tableau 1).

**Tableau 1**

| | **Hydrocarbures totaux (ppm) en fonction du temps** | | | | |
|---|---|---|---|---|---|
| **Durée** | **Initiale** | **2 mois** | **4 mois** | **6 mois** | **8 mois** |
| **Zone 1** | 62028 | 27765 | 5031 | 939 | 114 |

Huit mois plus tard, les terres alors décontaminées ont été abandonnées sur le site pour réaliser un espace vert

### Exemple 2

Le sol traité était l'objet d'une contamination sévère de solvants de peintures. Après une première intervention sur le site avec un tambour oléophile (voir brevet français publié sous le n° 2528412) pour éliminer les hydrocarbures libres de surface, la teneur moyenne du sol à traiter en hydrocarbures totaux était de 965 mg/kg, dont 57 mg/kg de toluène. Le contrôle bactérien attestait la présence de bactéries aérobies adaptées à la biodégradation des aromatiques légers (3 10⁸ bactéries par gramme de sol).

Les conditions du traitement ont été arrêtées ainsi :
- pulvérisation du nutriment oléophile Inipol EAP22 à la dose de 100 g/m² ;
- ajout de paille à raison de 1 % en volume ;
- ajout d'un nutriment hydrophile de composition N/P/K 18/1/10, commercialisé par CECA S.A. sous le nom d'Inipol®SP1, à la dose de 500 g/m³ ;
- malaxage avec trois reprises des terres.

Le matériau traité a été excavé et disposé en 10 andains de 5 mètres de large sur 50 mètres de long et 1,5 mètre de haut déposés sur une zone étanche. Des drains perforés ont été installés au coeur des andains pour parfaire l'aération du matériau. Son évolution a été la suivante :

| **Durée** | **Initiale** | **1 mois** | **3 mois** | **6 mois** |
|---|---|---|---|---|
| **HC totaux (ppm)** | 965 | 895 | 382 | 48 |
| **Toluène (ppma** | 57 | < seuil | < seuil | < seuil |

Après 6 mois, le sol considéré comme décontaminé à été réutilisé et épandu sur le site.

Le procédé s'applique à des terres où la présence de bactéries aptes à la biodégradation des hydrocarbures est attestée. Le procédé selon l'invention s'applique également à des sols stériles contaminés sous réserve d'un traitement préalable par inoculation/ensemencement bactérien.

Le mode de réhabilitation des sols est encore applicable à des contaminations par des compositions chimiques, par exemple des peintures ou d'autres composés organiques biodégradables.

## Revendications

1. Procédé de réhabilitation de sols pollués par des hydrocarbures dans lequel on favorise l'action d'une flore microbienne endogène apte à la dégradation des hydrocarbures par addition des nutriments déficitaires par rapport à un rapport idéal de développement bactérien C/N/P de 100/20/1, caractérisé en ce qu'on apporte au sol à traiter
- un nutriment du type N/P sous une forme oléophile, constitué d'une microémulsion du type solution de sels N/P dans un solvant hydrocarboné lipomiscible.
- un nutriment du type N/P/K sous forme hydrophile, constitué de granulés de sels N/P/K entourés d'une barrière antidiffusion.
- un agent texturant d'aération.

2. Procédé selon la revendication 1, caractérisé en ce que les grains sont d'une taille telle que l'on compte de 100 à 200 grains par gramme.

3. Procédé selon la revendication 2, caractérisé en ce que les grains sont fortement colorés.

4. Procédé selon la revendication 1, caractérisé en ce que l'agent texturant d'aération est de la paille.

5. Procédé selon la revendication 1, caractérisé en ce que l'on exécute :
- a) une pulvérisation du nutriment oléophile sur la surface du sol à traiter, puis
- b) un épandage de l'agent texturant et du nutriment hydrophile, puis
- c) un malaxage du sol traité jusqu'à mélange intime de tous ses constituants.

6. Procédé selon la revendication 5, caractérisé en ce que la phase b) de l'opération est différée de 0 à 7 jours.

7. Procédé selon l'une ou l'autre des revendications 5 ou 6, caractérisé en ce que le sol traité est abandonné sur place jusqu'à ce que la teneur des hydrocarbures s'abaisse au niveau de concentration désiré.

8. Procédé selon l'une ou l'autre des revendications 6 ou 7, caractérisé en ce que le sol traité est excavé et abandonné en andains jusqu'à ce que la teneur des hydrocarbures s'abaisse au niveau de concentration désiré.

## Patentansprüche

1. Verfahren zur Sanierung (Dekontamination) von mit Kohlenwasserstoffen verschmutzten Böden, bei dem man die Wirksamkeit einer zum Abbau von Kohlenwasserstoffen geeigneten, endogenen mikrobiologischen Flora durch Zugabe von bezüglich eines bakteriellen C/N/P-Entwicklungsidealverhältnisses von 100/20/1 defizitären Nutrimenten (Nährstoffen) begünstigt, dadurch gekennzeichnet, daß man dem zu behandelnden Boden zusetzt:
- ein Nutriment vom N/P-Typ in oleophiler Form, bestehend aus einer Mikroemulsion vom Typ Lösung von N/P-Salzen in einem fettmischbaren Kohlenwasserstofflösungsmittel;
- ein Nutriment vom N/P/K-Typ in hydrophiler Form, bestehend aus Granulat von N/P/K-Salzen, welche von einer Isolierschicht gegen Diffusion umgeben sind;
- ein strukturveränderndes Mittel zur Belüftung.

2. Verfahren nach Anspruch 1, dadurch gekennzeichnet, daß die Körner eine derartige Größe aufweisen, daß man 100 bis 200 Körner pro Gramm zählt.

3. Verfahren nach Anspruch 2, dadurch gekennzeichnet, daß die Körner stark gefärbt sind.

4. Verfahren nach Anspruch 1, dadurch gekennzeichnet, daß das strukturverändernde Mittel zur Belüftung Stroh ist.

5. Verfahren nach Anspruch 1, dadurch gekennzeichnet, daß
a) man das oleophile Nutriment auf der Oberfläche des zu behandelnden Bodens zerstäubt, anschließend
b) das strukturverändernde Mittel und das hydrophile Nutriment mittels Düngerstreuung ausbringt, dann
c) den behandelten Boden durcharbeitet, bis alle seine Bestandteile innig vermischt sind.

6. Verfahren nach Anspruch 5, dadurch gekennzeichnet, daß der Schritt b) des Verfahrens sich nach 0 bis 7 Tagen anschließt.

7. Verfahren nach Anspruch 5 oder 6, dadurch gekennzeichnet, daß man den behandelten Boden an Ort und Stelle läßt, bis sich der Kohlenwasserstoffgehalt auf das gewünschte Konzentrationsniveau abgesenkt hat.

8. Verfahren nach Anspruch 6 oder 7, dadurch gekennzeichnet, daß man den behandelten Boden aushebt und in Schwaden beläßt, bis sich der Kohlenwasserstoffgehalt auf das gewünschte Konzentrationsniveau abgesenkt hat.

## Claims

1. Process for the rehabilitation of soils polluted by hydrocarbons, in which the action of an endogenous microbial flora capable of degrading hydrocarbons is promoted by addition of nutrients that are in deficit relative to an ideal C/N/P bacterial growth ratio of 100/20/1, characterized in that the soil to be treated is supplied with
- a nutrient of the N/P type in oleophilic form, consisting of a microemulsion of the type: solution of N/P salts in a fat-soluble hydrocarbon solvent.
- a nutrient of the N/P/K type in hydrophilic form, consisting of granules of N/P/K salts surrounded by an anti-diffusion barrier.
- a texturing agent for aeration.

2. Process according to Claim 1, characterized in that the grains are of a size such that there are from 100 to 200 grains per gram.

3. Process according to Claim 2, characterized in that the grains are highly coloured.

4. Process according to Claim 1, characterized in that the texturing agent for aeration is straw.

5. Process according to Claim 1, characterized in that the following are performed:
- a) the oleophilic nutrient is sprayed onto the surface of the soil to be treated, then
- b) the texturing agent and the hydrophilic nutrient are spread, and then
- c) the treated soil is mixed until all of its constituents are intimately mixed.

6. Process according to Claim 5, characterized in that phase b) of the operation is deferred for 0 to 7 days.

7. Process according to either of Claims 5 and 6, characterized in that the treated soil is left in place until the hydrocarbon content has lowered to the desired level of concentration.

8. Process according to either of Claims 6 and 7, characterized in that the treated soil is dug out and left in swaths until the hydrocarbon content has lowered to the desired level of concentration.
